(19) **Europäisches Patentamt European Patent Office Office européen des brevets**

(11) **EP 4 181 966 B1**

(12) **EUROPEAN PATENT SPECIFICATION**

(45) Date of publication and mention of the grant of the patent:
**26.06.2024 Bulletin 2024/26**

(21) Application number: **21772850.0**

(22) Date of filing: **20.07.2021**

(51) International Patent Classification (IPC):
**A61L 2/00** *(2006.01)* **A61L 2/12** *(2006.01)*
**A61L 9/18** *(2006.01)* **A61L 2/24** *(2006.01)*

(52) Cooperative Patent Classification (CPC):
**A61L 2/0064; A61L 2/12; A61L 2/24; A61L 9/18;**
A61L 2202/14; A61L 2202/25; A61L 2209/111

(86) International application number:
**PCT/IT2021/000036**

(87) International publication number:
**WO 2022/018772 (27.01.2022 Gazette 2022/04)**

(54) **MICROWAVE DISINFECTION SYSTEM AND METHOD**

MIKROWELLENDESINFEKTIONSSYSTEM UND -VERFAHREN

SYSTÈME ET PROCÉDÉ DE DÉSINFECTION PAR MICRO-ONDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.07.2020 EP 20186787**

(43) Date of publication of application:
**24.05.2023 Bulletin 2023/21**

(73) Proprietor: **Elettronica S.p.A.**
**00131 Roma (IT)**

(72) Inventors:
• **PASCULLI, Nicola**
**00131 Roma (IT)**
• **MANNA, Antonio**
**00131 Roma (IT)**
• **TAFUTO, Antonio**
**00131 Roma (IT)**

(74) Representative: **Studio Torta S.p.A.**
**Via Viotti, 9**
**10121 Torino (IT)**

(56) References cited:
WO-A2-2004/069288 AU-A4- 2020 101 948
CN-A- 111 481 711 US-A1- 2021 085 814
US-B1- 10 561 751

**Description**

## TECHNICAL FIELD OF THE INVENTION

The present invention relates, in general, to a system and a method for microwave disinfection.

More specifically, the present invention relates to a system configured to destroy and/or inactivate pathogens (i.e. microorganisms that cause or may cause, or are responsible for causing, a disease in the human body, such as, for example, viruses, coronaviruses, viroids, germs, bacteria, mycetes (i.e. fungi), moulds, protozoa, prions, etc.) by microwave irradiation. In addition, the present invention also relates to a related microwave disinfection method.

## BACKGROUND

Viral and/or bacterial infections are known to cause significant mortality, particularly in elderly patients and/or with impaired immune systems.

Viruses and bacteria survive on different environmental surfaces for several hours and some of them, even for days, thereby the risk of transmission from hands to surfaces and vice versa is very high.

This adds to the fact that for particularly infectious pathogens, such as SARS-CoV-1, SARS-CoV-2, influenza viruses or enteroviruses, transmission routes include respiratory transmission by droplets and aerosols.

In order to reduce the risk of infection by viruses and bacteria through respiratory transmission, cleaning and disinfecting thoroughly the surfaces and objects in an environment is not enough. Inactivation techniques must be adopted that act on the molecular structure of pathogens, making them unable to reproduce, thus significantly reducing the risk of infections.

Known disinfection techniques involve the use of various technologies, sometimes used in combination with each other, providing high levels of effectiveness in inactivating pathogens, but which nevertheless have several limitations related to the fact that their disinfection effectiveness is temporary, that they potentially release substances/elements that are harmful to the human body and that cannot be used with people being present.

For example, a well-known disinfection technique is based on the so-called Ultraviolet Germicidal Irradiation (UVGI), which uses ultraviolet (UV) light with wavelengths in the UV-C band (i.e. with wavelengths between 100 nm and 280 nm), which modifies the DNA or RNA of microorganisms and thus prevents them from reproducing or being harmful.

Unfortunately, however, UV-C radiation is harmful to humans and other forms of life. Therefore, the use of UVGI devices requires the implementation of specific safety measures (such as the use of hazard warnings and/or warning devices/systems, operating equipment, risk information, etc.) and, often, also the use of personal protective equipment (PPE) such as eye protection, gloves, etc.

In addition, disinfection techniques based on the use of ozone are also known. As known, ozone (Os) is formed from oxygen molecules ($O_2$) in the presence of electrical discharges. Ozone is a powerful oxidising gas with antibacterial properties that spreads evenly throughout the environment to be treated, oxidising all organic compounds.

Research carried out on the effect of exposure to ozone has shown that ozone causes various types of effects affecting the respiratory tract, in particular it may:

- irritate the respiratory tract;
- reduce pulmonary function; and
- worsen asthma and other respiratory diseases.

In summary, it can be stated that the same chemical properties that allow ozone in high concentrations to react with organic material outside the human body, also make it react with similar organic material inside the human body, with consequent potential risk/damage for the human organism.

In general, therefore, it is not possible to remain in an environment during, and also for some time after, an ozone-based disinfection treatment and, before being able to occupy the room again, it must be ventilated (which is not always possible to do).

For disinfection purposes, photocatalysis technology is also well known. It is based on the activation of a compound called a photocatalyst on the surface in the presence of light, which is able to generate radicals and reactive compounds that interact with organisms or chemical substances in the environment, which may come into contact with the photocatalytic surface either directly (by deposition) or by proximity (in the laminar layer of air near the surface).

Photocatalysis may induce degradation in the case of simple compounds (proteins and DNA), an inhibiting effect in the case of viruses and bacteria.

Currently, photocatalytic technologies have a number of drawbacks that make them difficult to be used, including low yield, the widespread need for UV radiations and the possibility of releasing potentially harmful partial reaction compounds.

For example, the most commonly studied photocatalyst, titanium dioxide, is only activated when lighted with UV radiation, which is typically not available in sufficient amounts indoors, thus requiring the use of specific artificial UV sources.

US 10 561 751 B1 discloses a sterilization assembly of a mobile device that includes a bacteria detection device, a microwave sterilization device, and a processor. The bacteria detection device detects a bacterial count on the mobile device by emitting light waves and receiving reflected light waves and analyzing a wavelength change in the reflected light waves. The microwave sterilization device emits microwaves to kill the bacteria. The processor sends a detection signal to the bacteria detection device to control the bacteria detection device to detect the bacterial count, and sends a sterilization signal to the microwave sterilization device to control the microwave sterilization device to emit the microwaves. The bacteria detection device sends detection feedback information to the processor. The microwave sterilization device sends sterilization feedback information to the processor.

## OBJECT AND SUMMARY OF THE INVENTION

In the light of the foregoing, the Applicant perceived the need to carry out a thorough research in order to try to develop an innovative disinfection system/device capable of overcoming or alleviating, at least in part, the disadvantages and limitations of known disinfection technologies, thereby conceiving the present invention.

Therefore, a general object of the present invention is to provide an innovative disinfection technology that is able to overcome or alleviate, at least in part, the advantages and limitations of the currently known disinfection technologies.

Furthermore, a specific object of the present invention is to provide an innovative system which is capable of destroying and/or inactivating pathogens in real time and which may also be used with people being present without causing harmful or detrimental effects to the human body.

These and other objects are achieved by the present invention in that it relates to a microwave disinfection system and a related microwave disinfection method, according to what defined in the appended claims.

In particular, the present invention relates to a microwave disinfection system configured to destroy or inactivate in real time one or more pathogens by one or more microwave irradiations, wherein said microwave disinfection system comprises a microwave disinfection device including:

- a control section including electronic control means and storage means; and
- a microwave irradiation section controlled by the electronic control means and configured to emit microwave signals.

Furthermore, the microwave disinfection system further comprises human-machine interface means connected to the control section and configured to allow a user to configure the control section by setting and storing in the storage means one or more predefined microwave irradiation parameters related to one or more predefined microwave disinfection treatments against one or more pathogens.

The electronic control means are configured to operate the microwave irradiation section on the basis of the predefined microwave irradiation parameter(s) stored in the storage means so that said microwave irradiation section performs the predefined microwave disinfection treatment(s) against said pathogen(s).

The microwave irradiation section is configured to, when operated by the electronic control means, perform one or more microwave irradiations based on said predefined microwave irradiation parameter(s).

The human-machine interface means are configured to allow a user to set and store in the storage means, for a specific pathogen, one or more respective predefined microwave irradiation parameters related to one or more respective predefined microwave disinfection treatments against said specific pathogen.

Furthermore, the present invention also relates to a microwave disinfection method which is based on the use of said microwave disinfection system and which comprises:

- performing microwave irradiation tests of one or more pathogens to determine one or more microwave irradiation parameters related to one or more microwave disinfection treatments against said pathogen(s);
- setting and storing in the storage means, by means of the human-machine interface means, the determined microwave irradiation parameter(s); and
- using the microwave disinfection device to perform said microwave disinfection treatment(s) against the pathogen (s) .

Additionally, the microwave disinfection method further comprises:

- performing microwave irradiation tests of a specific pathogen to determine one or more respective microwave irradiation parameters related to one or more respective microwave disinfection treatments against said specific pathogen;

- setting and storing in the storage means, by means of the human-machine interface means, the respective microwave irradiation parameter(s) determined for the specific pathogen; and
- using the microwave disinfection device to perform said microwave disinfection treatment(s) against said specific pathogen.

## BRIEF DESCRIPTION OF THE DRAWINGS

For a better understanding of the present invention, some preferred embodiments (provided purely by way of explanatory, but absolutely not limiting, let alone binding example) will now be shown with reference to the accompanying drawings (not to scale), wherein:

- Figure 1 schematically shows a microwave disinfection system according to an embodiment of the present invention;
- Figures 2-6 schematically show different embodiments of the microwave disinfection system shown in Figure 1; and
- Figure 7 shows experimental results related to in vitro inactivation of SARS-CoV-2 using microwave inactivation treatment.

## DESCRIPTION OF PREFERRED EMBODIMENTS OF THE INVENTION

The following description is provided to enable a person skilled in the art to make and use the invention. Various modifications to the embodiments set forth will be immediately clear to the persons skilled in the art and the general principles herein disclosed may be applied to other embodiments and applications without, however, departing from the scope of protection of the present invention as defined in the appended claims.

Therefore, the present invention should not be understood as limited to the sole embodiments described and shown, but it must be given the widest scope of protection in accordance with the characteristics defined in the appended claims.

The present invention relates, first of all, to a microwave disinfection system, in particular a system configured to destroy and/or inactivate in real time pathogens (i.e. microorganisms that cause or may cause, or are responsible for causing, a disease in the human body, such as, for example, viruses, coronaviruses, viroids, germs, bacteria, mycetes (i.e. fungi), moulds, protozoa, prions, etc.) by microwave irradiation and related resonant microwave absorption by the pathogens, wherein said microwave disinfection system may also be used with people being present without causing harmful effects on the human body.

Furthermore, the present invention also relates to a microwave disinfection method based on the use of said microwave disinfection system.

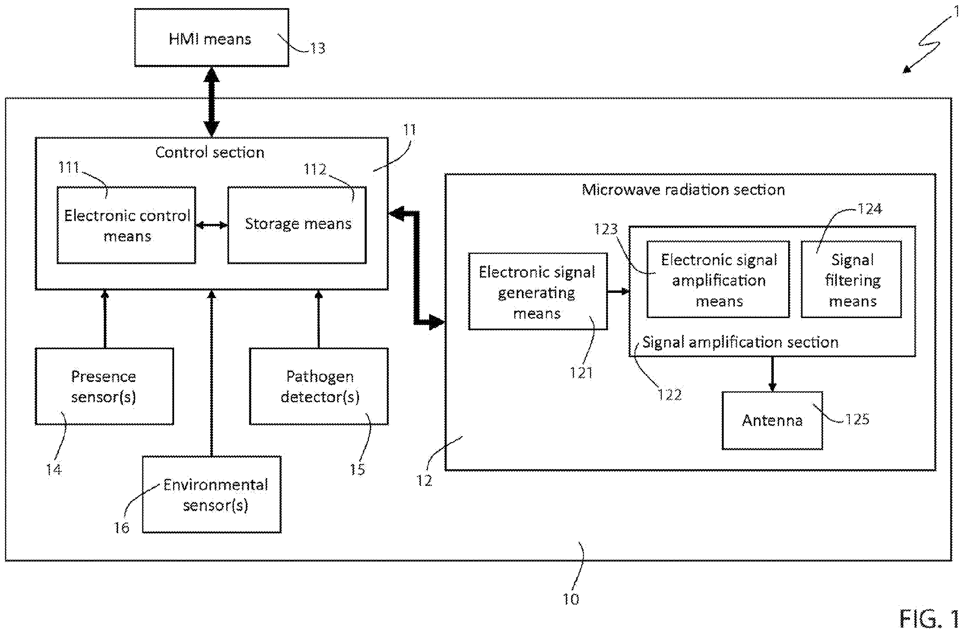

For a better understanding of the present invention, an example of a high-level functional architecture of a microwave disinfection system (entirely denoted by 1) according to a preferred (but by no means limiting, let alone binding) embodiment of the present invention is schematically shown (in particular by means of a functional block diagram) in Figure 1.

In particular, the microwave disinfection system 1 comprises a microwave disinfection device 10 including:

- a control section 11 (for instance, conveniently implemented in the form of one or more respective electronic boards) including electronic control means 111 and storage means 112 (for instance, conveniently implemented in the form of one or more setup storages); and
- a microwave irradiation section 12 which preferably includes

  - electronic signal generating means 121 (for instance, conveniently made in the form of one or more respective electronic boards) controlled by the electronic control means 111,
  - a signal amplification section 122 which, in use, is commanded by the electronic signal generating means 121 and is controlled by the electronic control means 111 and which preferably includes electronic signal amplification means 123 and signal filtering means 124, and
  - an antenna 125 which, in use, is commanded by the signal amplification section 122.

Furthermore, the microwave disinfection system 1 also comprises human-machine interface (HMI) means 13 which, in the example shown in Figure 1, are external to the microwave disinfection device 10 and are remotely connected, preferably in wireless mode, to the control section 11.

More generally, HMI means 13 may be connected to the control section 11 in a wired or wireless mode, for example via a connection based on Universal Serial Bus technology (USB), or via one or more networks based on Internet Protocol (IP), conveniently the Internet network, and/or via one or more cellular telephone networks (e.g., GSM, GPRS, UMTS, HSPA, LTE, 4.5G, 5G, etc.) and/or one or more local, home, business, public, private area networks, or via Bluetooth, etc.

Such HMI means 13 may be conveniently implemented by means of a PC, laptop, tablet, smartphone, smartwatch, etc.

According to an embodiment alternative to that shown in Figure 1, HMI means 13 may, instead, be conveniently integrated into the microwave disinfection device 10, e.g. they may be conveniently implemented in the form of one or more displays (such as of the touchscreen type) and/or one or more User Interface (UI) devices of the hard and/or soft key type.

The control section 11 may be configured by a user by means of the HMI means 13 by:

- setting one or more predefined microwave irradiation parameters related to one or more predefined microwave disinfection treatments against one or more pathogens; and
- storing said predefined microwave irradiation parameters in the storage means 112.

In other words, HMI means 13 are configured to allow a user to set up and store in the storage means 112 one or more predefined microwave irradiation parameters related to one or more predefined microwave disinfection treatments against one or more pathogens, thereby configuring the control section 11 to perform said predefined microwave disinfection treatment(s) against said pathogen(s) by means of the microwave irradiation section 12.

Preferably, HMI means 13 are configured to allow a user to set and store in the storage means 112, for a specific pathogen (conveniently, for each pathogen of a plurality of given pathogens), one or more respective predefined microwave irradiation parameter(s) related to one or more respective predefined microwave disinfection treatments against said specific pathogen, wherein said respective predefined microwave irradiation parameter(s) may conveniently include one or more of the following parameters related to microwave inactivation of said specific pathogen:

- one or more predefined microwave irradiation waveforms;
- one or more predefined microwave irradiation wavelengths (i.e. one or more predefined frequencies) and/or one or more predefined wavelength bands (i.e. one or more predefined frequency bands) of microwave irradiation;
- one or more predefined microwave irradiation timing parameters (e.g., in terms of duration of each individual microwave irradiation and frequency of repetition of microwave irradiations);
- one or more predefined microwave irradiation powers.

Conveniently, HMI means 13 are configured to store in the storage means 112, for the specific pathogen (conveniently, for each pathogen of said given pathogens), a respective library containing said respective predefined microwave irradiation parameter(s).

The electronic control means 111 are, therefore, configured to operate, i.e. to control the operation of, said microwave irradiation section 12 (in particular to operate, i.e. to control the operation of said electronic signal generating means 121 and said signal amplification section 122) on the basis of said predefined microwave irradiation parameter(s) stored in the storage means 112 so that said microwave irradiation section 12 performs said predefined microwave disinfection treatment(s) against said pathogen (s) .

More specifically, the microwave irradiation section 12 is configured to perform, when operated by the electronic control means 111, one or more microwave irradiations based on said predefined microwave irradiation parameter(s), or to emit microwave signals in accordance with said predefined microwave irradiation parameter(s) (e.g., with the predefined waveform(s), and/or the predefined wavelength or frequency(s), and/or predefined wavelength or frequency(s), and/or the predefined wavelength or frequency band(s), and/or the predefined timing parameter(s), and/or the predefined power(s)).

As shown in Figure 1, the microwave disinfection device 10 preferably also comprises one or more presence (or proximity) sensors 14 (for instance, conveniently based on infrared - IR technology) configured to:

- detect the presence of one or more persons near the microwave disinfection device 10 (e.g., within a predefined distance from the latter), or in a given environment in which said microwave disinfection device 10 is installed/arranged; and
- signal the presence of one or more persons to the electronic control means 111, which are preferably configured to operate the microwave irradiation section 12 when the presence sensor(s) 14 detects the presence of one or more persons.

In addition, the microwave disinfection device 10 preferably also comprises one or more pathogen 15 detectors configured to:

- detect the presence of one or more pathogen(s) (conveniently, one or more of said given pathogens); and
- signal the presence of the one or more pathogens to the electronic control means 111, which are preferably configured to operate the microwave irradiation section 12 based on the predefined microwave irradiation parameter(s) stored in the storage means 112 (conveniently, based on the respective predefined microwave irradiation parameter(s) stored in the storage means 112 for the pathogen(s) detected by the pathogen detector(s) 15).

Such pathogen detector(s) 15 may be conveniently implemented by means of one or more sensors of the CRISPR type (Clustered Regularly Interspaced Short Palindromic Repeats), such as the field-effect transistors disclosed in Reza Hajian et al, "Detection of unamplified target genes via CRISPR-Cas9 immobilized on a graphene field-effect transistor", Nature Biomedical Engineering, vol. 3, pp. 427-437, 25 March 2019, or the synthetic biological sensors disclosed in Peter. Q. Nguyen et al, *"Wearable materials with embedded synthetic biology sensors for biomolecule detection",* Nature Biotechnology, 28 June 2021.

The microwave disinfection device 10 may also conveniently comprise one or more environmental sensors 16 (e.g., a temperature sensor and/or a pressure sensor and/or a humidity sensor and/or one or more detectors of pollutant/harmful elements/substances, etc.) configured to send respective environmental monitoring data to the electronic control means 111, which are conveniently configured to control the operation of the microwave irradiation section 12 based also on the environmental monitoring data received from the environmental sensor(s) 16.

Conveniently, the microwave disinfection device 10 also comprises a power supply section (not shown in Figure 1 for simplicity) for supplying electrical power to the control section 11 (in particular, to the electronic control means 111 and the storage means 112), the microwave irradiation section 12 (in particular, to the electronic signal generating means 121 and the signal amplification section 122), the presence sensor(s) 14, the pathogen detector(s) 15 and the environmental sensor(s) 16.

Conveniently:

- the electronic control means 111 may be, for example, implemented by means of a microcontroller which has two cores with WLNA, Bluetooth, SPI, I2Cbus interfaces so as to be able to communicate with different mobile devices and/or external HMIs, to program the various logical functions of the microwave disinfection device 10 and to manage the data exchange between the sensors/detectors 14, 15, 16 and other components of the microwave disinfection device 10;
- the electronic signal generating means of 121 may, for example, comprise a programmable synthesised frequency generator capable of generating a variable frequency signal, for example between 6 and 18 GHz (e.g., through the use of one or more tunable local oscillators, one or more mixers, one or more digital-to-analogue converters, etc.) - the management of the time sequence of the generated frequencies may, for example, be performed by appropriate programming of the electronic control means 111;
- the electronic signal amplification means 123 are configured to amplify the signal provided at the output of the electronic signal generating means 121, for example to transmit by means of the antenna 125 a power signal up to 10 W at continuous wave (CW) - the electronic signal amplification means 123 may be, for example, implemented by means of a variable radio frequency (RF) attenuator and two amplifiers of medium and high power respectively - the correct attenuation values may be set by means of the electronic control means 111 and it is possible to control switching on the amplifiers so that the correct power values to be irradiated can be sent towards the antenna 125;
- the output signal from the signal amplification section 122 is sent via RF cable to the antenna 125 by means of which the electromagnetic signal is irradiated in a predefined coverage area and at predefined electric field levels;
- the sensors/detectors 14, 15, 16 are able to provide information to the electronic control means 111 which, by means of programmable logic, can manage different techniques for inactivating pathogens; furthermore, the information provided by the sensors/detectors 14, 15, 16 may also be sent to HMI means 13;
- the power supply section may be, for example, implemented by means of an alternating to direct current (AC/DC) converter and a series of DC/DC converters capable of transforming a 220V AC input supply voltage to the DC voltages required to power the various devices/components of the microwave disinfection device 10.

As explained above, the present invention also relates to a microwave disinfection method based on the use of the microwave disinfection system 1. Said microwave disinfection method comprises:

- performing microwave irradiation tests of one or more pathogens to determine one or more microwave irradiation parameters related to one or more microwave disinfection treatments against said pathogen(s);
- setting and storing in the storage means 112, by means of the HMI 13 means, the determined microwave irradiation parameter(s); and
- using the microwave disinfection device 10 to perform said microwave disinfection treatment(s) against the pathogen(s) .

Preferably, the microwave disinfection method comprises:

- performing microwave irradiation tests of a specific pathogen to determine one or more respective microwave irradiation parameters related to one or more respective microwave disinfection treatments against said specific pathogen;
- setting and storing in the storage means 112, by means of the HMI means 13, said respective microwave irradiation parameter(s) determined for the specific pathogen; and
- using the microwave disinfection device 10 to perform said microwave disinfection treatment(s) against said specific pathogen.

Conveniently, the microwave disinfection method comprises:

- performing microwave irradiation tests of a plurality of given pathogens to determine, for each of the given pathogens, one or more respective microwave irradiation parameters related to one or more respective microwave disinfection treatments against said pathogen of said plurality of given pathogens;
- setting and storing in the storage means 112, by means of HMI means 13, the microwave irradiation parameters determined for the given pathogens; and
- using the microwave disinfection device 10 to perform microwave disinfection treatments against said pathogens.

Conveniently, the respective microwave irradiation parameter(s) determined for the specific pathogen, or for each of the given pathogens, include/includes one or more of the following parameters:

- one or more microwave irradiation waveforms;
- one or more microwave irradiation wavelengths (i.e. one or more frequencies) and/or one or more microwave irradiation wavelength bands (i.e. one or more frequency bands);
- one or more microwave irradiation timing parameters;
- one or more microwave irradiation powers.

From the foregoing explanation, it derives that the microwave disinfection system 1 appears to be programmable and has air sanitization agnostic abilities by using microwaves, conveniently via the technique called Microwave Resonant Absorption (MRA) - in this regard, reference can be made, for example, to Yang SC. et al., "Efficient Structure Resonance Energy Transfer from Microwaves to Confined Acoustic Vibrations in Viruses", Scientific Reports 5, 18030, December 2015. The microwave disinfection system 1 is able to sanitise the air from pathogenic elements in real time (in this context the expression "in real time" may conveniently indicate a time period in the order of a few minutes) with a technique that does not require evacuation of the area submitted to the microwave disinfection treatment as the emission of the microwave signals will conveniently always be below a predefined safety threshold (e.g., in accordance with the maximum radiated electric field strength to which a person may be subjected as provided by the International Commission on Non-Ionizing Radiation Protection (ICNIRP) - "ICNRIP Guidelines for Limiting Exposure to Time-Varying Electric, Magnetic and Electromagnetic Fields (Up to 300 GHz)", Health Physics 74(4), p. 494-522, 1998).

The microwave disinfection system 1 may be conveniently dimensioned for disinfecting small to medium-sized, preferably confined areas (e.g. lifts, train/metro carriages, planes, medical premises, such as operating rooms, commercial premises, meeting rooms, offices, etc.). The microwave disinfection system 1 has the ability to be programmable as it is preferably based on a Software Defined Radio (SDR) architecture and it is possible to load the settings related to time, duration, power and frequency of microwave emission depending on the pathogens to be inactivated (i.e., each virus corresponds to a frequency of microwave signal coupling with its dipolar acoustic resonance). This means that the microwave disinfection system 1 is able to be programmed to contrast new pathogens (and their variants) once identified the characteristics of the optimal type of microwave transmission for their inactivation. It is thereby possible to create an "optimised pathogen-waveform inactivation microwave signal" library. This library may be updated on the microwave disinfection device 10 as new viruses or, more generally, new pathogens appear.

The microwave disinfection system 1 is preferably able to work in an "agnostic" manner, i.e. it is capable of carrying out an analysis of the air by means of the dedicated sensors/detectors 14, 15, 16 and, on the basis of this analysis, it is able to program itself to maximise the effectiveness of air disinfection through the use of a library with inactivation waveform types related to each specific pathogen.

The microwave disinfection device 10 may also be configured to work in two modes:

1) an active mode, i.e. it only activates when there are people in the room where it is placed in order to perform disinfection only when necessary, thus optimising energy consumption; and/or

2) a timed mode, i.e. it may be conveniently configured for periodically generating programmable and automatic microwave signals for emitting microwaves even without people.

The microwave disinfection device 10 is also able to connect to an external control unit by means of a physical interface (via USB or Ethernet or other) or wirelessly (Bluetooth, or other type of RF connection) in order to be programmed or to exchange data and/or information regarding the status of the device or the analysis of the air in the agnostic mode by means of a special software application (also known simply as an app).

As explained above (and as shown in Figure 1), the microwave disinfection device 10 may conveniently include:

- a power supply section;
- a backplane to which the digital and analogue parts, the power supply and all external connections are connected;
- one or more digital processing boards which is/are the heart of the SDR structure of the system, which may be programmed and which may generate the analogue baseband signals;
- an analogue microwave part which performs the up-conversion and amplification of the signal; and
- an antenna 125, preferably an Ultra Wide Band (UWB) antenna, capable of irradiating the generated microwave signal.

The composition of the microwave disinfection system 1 may also be conveniently optimised according to the type of environment in which it operates. In fact, depending on the type of installation, in addition to the level of power and frequency, it is possible to use an antenna with a different type of beam in order to best irradiate and thus maximise the disinfection process. The shape and directivity of the beam may be programmed locally (e.g. by pre-programming) or remotely.

Furthermore, the microwave disinfection system 1 may conveniently also perform a multispectral-type treatment as it may also conveniently have a section capable of sucking in air and performing a UVGI type disinfection treatment inside the microwave disinfection device 10 by means of a UV-C lamp.

In view of the above, it is important for the present invention to study the optimal frequencies and waveforms for activating the acoustic resonance of the specific pathogen by energy transfer from the microwave signal. In this context, it is important to study the physical structure and electrostatic signature of the pathogens to be inactivated. Following this analysis, it is then possible to populate the "library" of the microwave disinfection system 1 (e.g., with the suitable waveforms, exposure times, power required for the specific microorganism, etc.).

The ability of the microwave disinfection system 1 to be "agnostic" may conveniently include the use of sensors 15 so that the pathogens of interest (ideally, all pathogens) may be detected.

The idea of creating a system for inactivating pathogens using the principle of resonant microwave absorption rises from the need to create a tool that, in addition to guaranteeing effective real-time disinfection of either air or surfaces, may also be used with people being present in closed or semi-closed environments.

The operation of the system according to the present invention is based on the ability to inactivate pathogens by the effect of dipolar coupling between microwave electromagnetic waves and Confined Acoustic Vibrations (CAV) . As known, in fact, Microwave Resonant Absorption (MRA) occurs when the confined acoustic vibration shifts the charges of microorganisms and changes their dipole moments.

The physical phenomenon attributed to the interaction between microwaves and CAVs results in the activation of the elastic resonance of the particle through coupling with acoustic vibration modes. The direct consequence of the resonant oscillation is the molecular fracture of the particle with the consequent inactivation of its reproduction processes.

The microwave disinfection system 1 shown in Figure 1 and described in detail above, makes it possible to obtain different embodiments of said system suitable for different operational scenarios and/or usable for different applications, i.e. so that they may be easily installed in environments with different characteristics and sizes and/or so that they may be advantageously exploited for different applications.

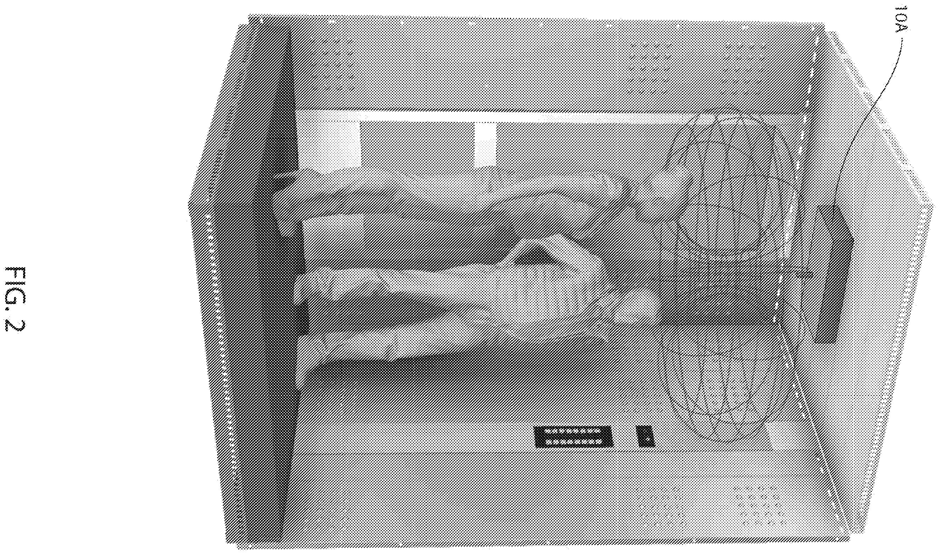

In this respect, Figures 2-4 schematically show (with parts removed for the clarity of illustration and parts schematised) three embodiments of the microwave disinfection system 1, more specifically the microwave disinfection device 10, namely:

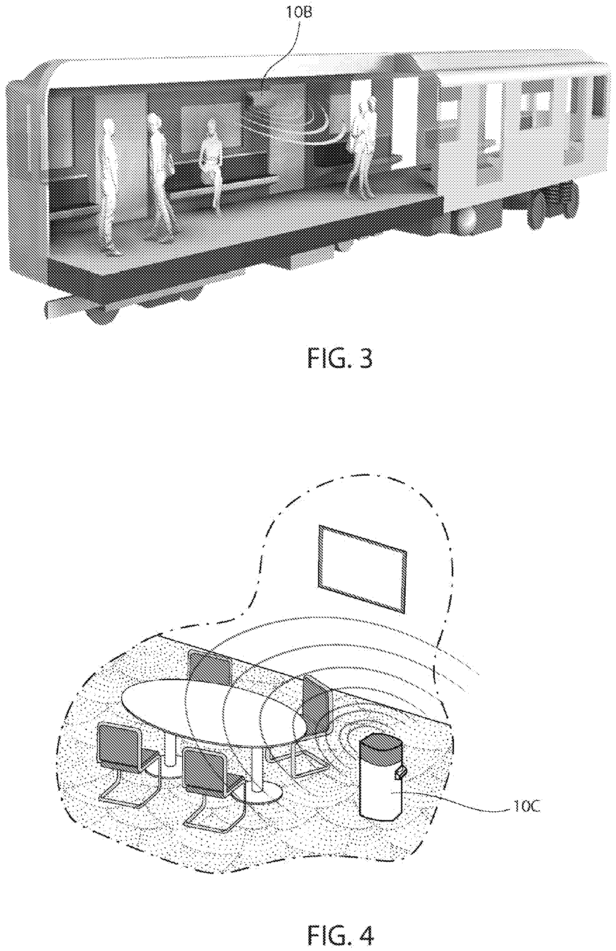

- a ceiling-mounted version (Figure 2, where 10A denotes the microwave disinfection device 10 and is installed on the ceiling of a lift);
- a wall-mounted version (Figure 3, where 10B denotes the microwave disinfection device 10 and is installed on a wall of a train carriage); and
- a floor version (Figure 4, wherein 10C denotes said microwave disinfection device 10 and is implemented as a device installed/placed on the floor, in particular on the floor of a meeting room, wherein said microwave disinfection device 10C could conveniently also be a device of a mobile/transportable type - for example, said microwave disinfection device 10C could conveniently have wheels).

More generally, the microwave disinfection system 1 (i.e. microwave disinfection device 10) may be advantageously used in any indoor environment (e.g. hospitals, canteens, offices, workplaces, schools, airports, stations, etc.).

A further application of the microwave disinfection system 1 is the disinfection/sanitisation of clothing made from non-metallic fabrics (e.g. cotton, wool, linen, etc.). In this case, the microwave disinfection system 1 can be conveniently configured to operate minimising the disinfection/sanitisation time as it does not require direct exposure of each individual face of the garments to be treated.

Indeed, the virus survival time on tissues is particularly important and represents a serious disinfection problem. It is known, for example, that SARS-CoV-2 virus particles have been detected up to one day after contamination (see Report by the Istituto Superiore di Sanità (ISS)): "Raccomandazioni ad interim sulla sanificazione di strutture non sanitarie nell'attuale emergenza COVID-19: superfici, ambienti interni e abbigliamento"- 15th May 2020). Recommended sanitisation methods provide using Medical Devices (Presidi Medico Chirurgici - PMC) or biocidal products. Both are chemicals that can lose their antiviral or antibacterial effectiveness over time, and the environmental impact and human health risks associated with their use must also be considered.

On the other hand, the microwave 1 disinfection system offers a solution to the above problems by ensuring the inactivation of the pathogens present on the fabrics, allowing them to be safely reused at the end of each disinfection/sanitisation cycle.

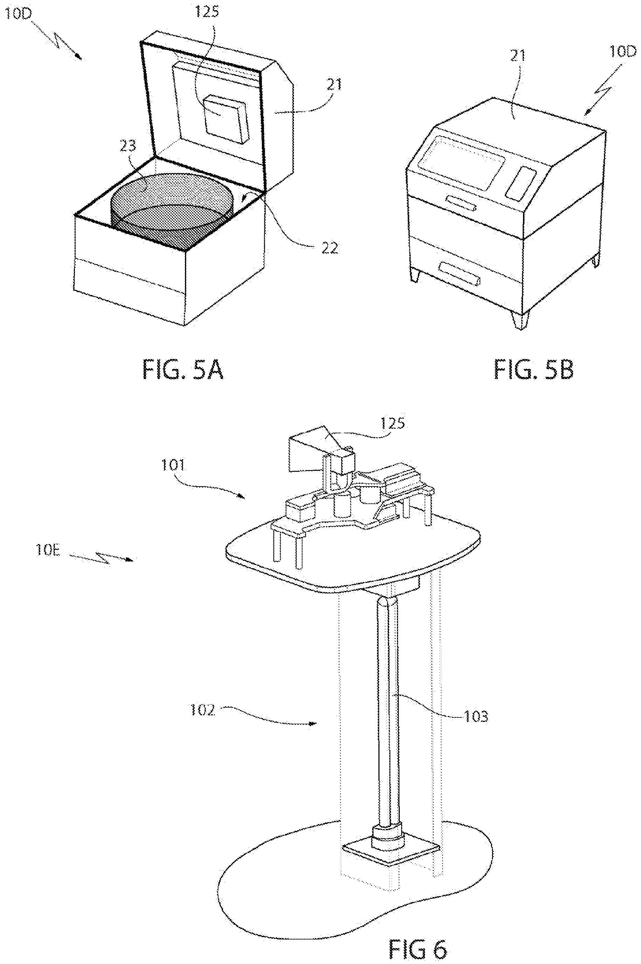

In this respect, Figures 5A and 5B schematically show an embodiment of the microwave disinfection system 1, more specifically of the microwave disinfection device 10, for the disinfection/sanitisation of clothing and/or garments (or, more generally, objects of any kind made of non-metallic fabrics).

In particular, in the example of Figures 5A and 5B, the microwave disinfection device 10 (which in said Figures 5A and 5B is denoted by 10D) is implemented in the form of a sort of top-loading washing machine, wherein:

- the control section 11 and the microwave irradiation section 12 are installed in an upper lid 21 which may be closed or opened, i.e. which is movable between

    - a first position in which it closes an inner compartment 22 in which a basket 23 is installed and
    - a second position in which it is raised, thus leaving the access to the inner compartment 22 and basket 23 open;

- the antenna 125 is installed on the upper lid 21 so that it faces the inner compartment 22 and the basket 23;
- when the upper lid 21 is open, i.e. raised (Figure 5A), it is possible to introduce into the basket 23 cloths or garments (e.g. suits, gowns, sheets, etc.) made of non-metallic fabrics intended to undergo microwave disinfection/sanitisation treatment;
- by contrast, when the upper lid 21 is closed (Figure 5B), the microwave disinfection/sanitisation treatment is performed on the cloths/garments present in the drum 23.

Furthermore, as mentioned above, the microwave disinfection system 1 may also conveniently have further pathogen inactivation technologies, such as ultraviolet lighting means configured to emit ultraviolet (UV) light, conveniently in the UV-C band, and/or means configured to perform an ozone-based disinfection treatment, and/or means based on photocatalysis technology.

In fact, within the microwave disinfection device 10 a further independent additional section may also be conveniently arranged in which the air present in the environment where the microwave disinfection device 10 is arranged is conveyed (e.g. by means of suction means) in order to submit the conveyed/sucked air to a disinfection/sanitisation/sterilisation treatment, for example of the UVGI type, and then reintroduce it into the environment.

This option offers the clear advantage of being able to simultaneously perform two different, mutually independent disinfection treatments using a single system/device. In addition, in the event of failure of one of the two subsystems (e.g. one for microwave disinfection and one for UVGI air disinfection/sanitisation/sterilisation), the other subsystem may continue to operate autonomously, allowing (e.g. health personnel) to safely complete the tasks they are involved in.

In this respect, Figure 6 shows schematically (with parts removed for clarity of illustration and parts schematised) an embodiment of the microwave disinfection system 1, more specifically the microwave disinfection device 10, to perform either microwave or UVGI-type disinfection treatments.

In particular, as shown in Figure 6, the microwave disinfection device 10 (which in said Figure 6 is denoted by 10E) includes:

- a first subsystem 101 that is arranged in an upper part of said microwave disinfection device 10E and is configured to perform one or more microwave disinfection treatments (in accordance with the foregoing); and
- a second subsystem 102 which is arranged in a lower part of said microwave disinfection device 10E, includes one or more UV lamps 103 and is configured to perform a UVGI-type disinfection/sanitization/sterilization treatment of the air conveyed through said lower part of the microwave disinfection device 10E.

In the light of the above, one of the main features of the microwave disinfection system 1 is the possibility of working on libraries; in fact, each different pathogen has its own characteristic resonance frequency determined by the physical structure and electrical signature of the particle.

It is then possible to create a library of frequencies to be used (and waveforms associated thereto) for each specific pathogen to be sanitised.

For example, the nominal resonance frequencies are reported in the following table (i.e., analytically calculated - see A. Barbora, R. Minnes, "Targeted antiviral treatment using Non-ionizing Radiation Therapy for SARS-CoV-2 and viral pandemics preparedness: Technique, methods and practical notes for Clinical Application", August 2020) whereby it is possible to maximise energy transfer for influenza A, EV71 and SARS-CoV-2 and thus achieve inactivation of these pathogens.

| Virus | Diameter (mm) | Resonance frequency (GHz) $f = \dfrac{2400}{2D}$ |
|---|---|---|
| Influenza A | 93 ± 5 (H D) | 12.9 ± 0.7 |
| | 100 (EM D) | 12 |
| EV71 | 35 ± 2 (H D) | 34 ± 2 |
| | 28.5 ± 1.5 (EM D) | 42 ± 2 |
| SARS-CoV-2 | 60 - 140 (EM D full range) | 8.5 - 20 |
| | 70 - 80 (EM D medium size) | 15 - 17 |

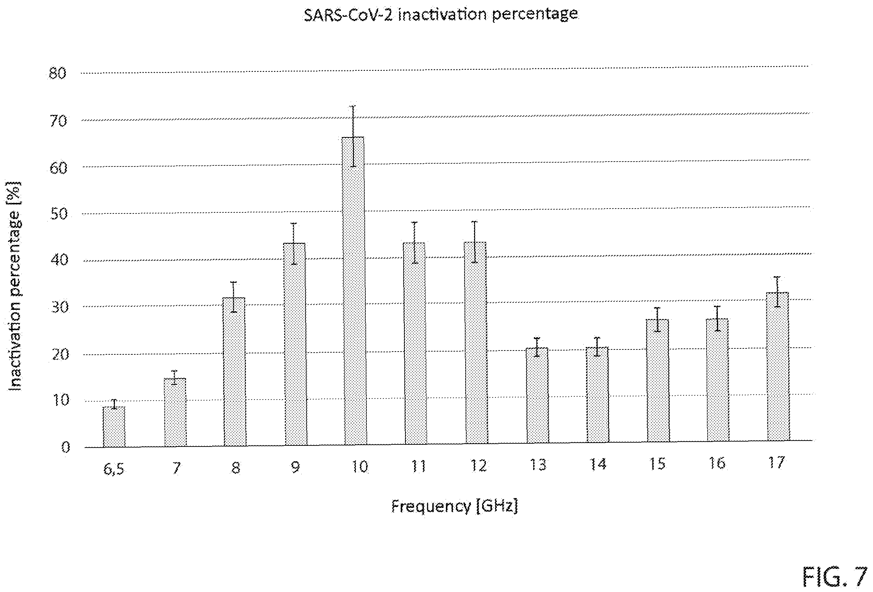

The Applicant carried out a first series of experimental in vitro inactivation tests of SARS-CoV-2 treated by microwave signal, the results of which are shown in Figure 7, where it can be noted that, the treatment of the viral culture sample being equal, a higher inactivation is obtained at 10 GHz.

The performance lower than 1 log is mainly due to the fact that the microwave signal is not able to penetrate aqueous solutions and therefore has, in this case, a limited effectiveness (in this regard, reference can be made, for example, to C. Wang et al., "Airborne disinfection using microwave-based technology: Energy efficient and distinct inactivation mechanism compared with waterborne disinfection", Journal of Aerosol Science, Vol. 137, 2019).

By contrast, the sanitisation rate becomes much higher (i.e. greater than 3 logs) when treatment is carried out in an aerosol. In this regard, the Applicant also carried out a second set of experimental aerosol inactivation tests of a solution containing SARS-CoV-2 using the following test parameters:

- 8-10 GHz band;
- 10 MHz steps;
- 3.2 seconds dedicated to each individual frequency for a total of 12 minutes of treatment;
- incident signal with a field amplitude of 100 V/m.

Inactivation was measured by cytopathic technique by comparing the residual titre of an aerosol-submitted but non-treated virus sample with an aerosol-submitted sample treated by microwave signal according to the set-up described above.

The above aerosol tests showed an inactivation percentage of SARS-CoV-2 equal to 83%.

From the foregoing disclosure, the several innovative characteristics and the innumerable technical advantages of the present invention are immediately evident for a person skilled in the art.

In particular, it is important to highlight the fact that the system according to the present invention is able to inactivate in real time a plurality of pathogens (viruses and bacteria of various shapes and dimensions) thanks to the possibility of identifying and activating, for each of them, a corresponding frequency suitable for activating the resonant effect.

The system according to the present invention represents the first possible mechanism for inactivating airborne viruses without restricting the presence of people, as the microwave energy required (i.e., the maximum intensity of the irradiated electric field) meets the guidelines set out by ICNIRP.

Furthermore, the system according to the present invention, unlike conventional disinfection methods, ensures continuous air disinfection and may operate effectively even in environments with poor or no air exchange.

The system according to the present invention may be programmed to selectively inactivate one or more pathogens according to the contamination characteristics of the installation site, conveniently also in multispectral mode by equipping the system with a UV-C lamp.

Finally, thanks to the use of one or more presence sensors, it is possible to program the operation of the system according to the present invention only with people being present, thus allowing to optimally manage energy consumption.

In conclusion, it is important to note that, while the above described invention refers in particular to very specific embodiments, it must not be intended as limited to such embodiments, including within its scope all the variants, modifications or simplifications covered by the enclosed claims.

**Claims**

1. Microwave disinfection system (1) configured to destroy or inactivate in real time one or more pathogens by means of one or more microwave irradiations, wherein said microwave disinfection system (1) comprises a microwave disinfection device (10) including:

   - a control section (11) including electronic control means (111) and storage means (112); and
   - a microwave irradiation section (12) controlled by the electronic control means (111) and configured to emit microwave signals;

   the microwave disinfection system (1) further comprising human-machine interface means (13) connected to the control section (11) and configured to allow a user to configure the control section (11) by setting and storing in the storage means (112) one or more predefined microwave irradiation parameters related to one or more predefined microwave disinfection treatments against one or more pathogens;

   wherein the electronic control means (111) are configured to operate the microwave irradiation section (12) based on the predefined microwave irradiation parameter(s) stored in the storage means (112) such that said microwave irradiation section (12) performs the predefined microwave disinfection treatment(s) against said pathogen(s);

   wherein the microwave irradiation section (12) is configured to, when operated by the electronic control means (111), perform one or more microwave irradiations based on said predefined microwave irradiation parameter(s);

   **characterized in that** the human-machine interface means (13) are configured to allow a user to set and store in the storage means (112), for a specific pathogen, one or more respective predefined microwave irradiation parameters related to one or more respective predefined microwave disinfection treatments against said specific pathogen.

2. The microwave disinfection system according to claim 1, wherein the human-machine interface means (13) are configured to allow a user to set and store in the storage means (112), for each pathogen of a plurality of given pathogens, one or more respective predefined microwave irradiation parameters related to one or more respective predefined microwave disinfection treatments against said pathogen of said plurality of given pathogens.

3. The microwave disinfection system according to claim 1 or 2, wherein the respective predefined microwave irradiation parameter(s) related to the specific pathogen or to each pathogen of said plurality of given pathogens include(s) one or more of the following parameters related to microwave inactivation of said specific pathogen or of said pathogen of said plurality of given pathogens:

   • one or more predefined microwave irradiation waveforms;
   • one or more predefined wavelengths or frequencies of microwave irradiation and/or one or more predefined bands of wavelength or frequency of microwave irradiation;
   • one or more predefined microwave irradiation timing parameters;
   • one or more predefined microwave irradiation powers.

4. The microwave disinfection system according to any claim 1-3, wherein the human-machine interface means (13) are configured to store in the storage means (112), for the specific pathogen or for each pathogen of the plurality of given pathogens, a respective library containing the respective predefined microwave irradiation parameter(s).

5. The microwave disinfection system according to any claim 1-4, wherein the microwave disinfection device (10) further comprises one or more pathogen detectors (15) configured to:

   - detect the presence of the specific pathogen or of one or more pathogens from the plurality of given pathogens;

and

- signal the presence of the specific pathogen or of one or more pathogens from the plurality of given pathogens to the electronic control means (111);

wherein the electronic control means (111) are configured to operate the microwave irradiation section (12) based on the respective predefined microwave irradiation parameter(s) stored in the storage means (112) for the specific pathogen or the pathogen(s) of the plurality of given pathogens detected by the pathogen detector(s) (15).

6. The microwave disinfection system according to any preceding claim, wherein the microwave disinfection device (10) further comprises one or more presence sensors (14) configured to:

   • detect the presence of one or more persons; and
   • signal the presence of one or more persons to the electronic control means (111);

wherein the electronic control means (111) are configured to operate the microwave irradiation section (12) when the presence sensor (s) (14) detect(s) the presence of one or more persons.

7. The microwave disinfection system according to any preceding claim, wherein the microwave disinfection device (10) further comprises one or more environmental sensors (16) configured to send respective environmental monitoring data to the electronic control means (111), and wherein said electronic control means (111) are configured to control operation of the microwave irradiation section (12) based also on the environmental monitoring data received from the environmental sensor(s) (16).

8. The microwave disinfection system according to any preceding claim, wherein the human-machine interface means (13) are:

   • external, and wired or wirelessly connected, to the microwave disinfection device (10); or
   • are integrated into said microwave disinfection device (10) .

9. The microwave disinfection system according to any preceding claim, wherein the microwave irradiation section (12) includes:

   • signal generating electronic means (121);
   • signal amplification electronic means (123); and
   • an antenna (125).

10. The microwave disinfection system of claim 9, wherein the microwave irradiation section (12) further includes signal filtering means (124).

11. Microwave disinfection method based on the use of the microwave disinfection system (1) as claimed in any one of the preceding claims, wherein said microwave disinfection method comprises:

   • performing microwave irradiation tests of one or more pathogens to determine one or more microwave irradiation parameters related to one or more microwave disinfection treatments against said pathogen(s);
   • setting and storing in the storage means (112), by means of the human-machine interface means (13), the determined microwave radiation parameter(s);
   • using the microwave disinfection device (10) to perform the microwave disinfection treatment(s) against the pathogen(s);

**characterized by** further comprising:

   • performing microwave irradiation tests of a specific pathogen to determine one or more respective microwave irradiation parameters related to one or more respective microwave disinfection treatments against said specific pathogen;
   • setting and storing in the storage means (112), by means of the human-machine interface means (13), the respective microwave irradiation parameter(s) determined for the specific pathogen; and
   • using the microwave disinfection device (10) to perform said microwave disinfection treatment(s) against said specific pathogen.

12. The microwave disinfection method according to claim 11, comprising:

> • performing microwave irradiation tests of a plurality of given pathogens to determine, for each of the given pathogens, one or more respective microwave irradiation parameters related to one or more respective microwave disinfection treatments against said pathogen of said plurality of given pathogens;
> • setting and storing in the storage means (112), by means of the human-machine interface means (13), the microwave irradiation parameters determined for the given pathogens; and
> • using the microwave disinfection device (10) to perform microwave disinfection treatments against said given pathogens.

13. The microwave disinfection method according to claim 11 or 12, wherein the respective microwave irradiation parameter(s) determined for the specific pathogen or for each of the given pathogens include(s) one or more of the following parameters related to microwave inactivation of said specific pathogen or said pathogen of the plurality of given pathogens:

> • one or more microwave irradiation waveforms;
> • one or more wavelengths or frequencies of microwave irradiation and/or one or more wavelength or frequency bands of microwave irradiation;
> • one or more microwave irradiation timing parameters;
> • one or more microwave irradiation powers.

## Patentansprüche

1. Mikrowellendesinfektionssystem (1), eingerichtet zum Zerstören oder Deaktivieren von Pathogenen über eine oder mehrere Mikrowellenbestrahlungen in Echtzeit, wobei das Mikrowellendesinfektionssystem (1) eine Mikrowellendesinfektionsvorrichtung (10) umfasst, die:

> - einen Steuerabschnitt (11) mit elektronischen Steuereinheiten (111) und Speichereinheiten (112); und
> - eine Mikrowellenbestrahlungseinheit (12), gesteuert über die elektronische Steuereinheit (11) zur Aussendung von Mikrowellensignalen umfasst;
> Ferner umfasst das Mikrowellendesinfektionssystem (1) eine Mensch-Maschine-Schnittstelle (12), die mit der Steuereinheit (11) verbunden und so konfiguriert ist, dass ein Benutzer die Steuereinheit (11) konfigurieren kann, indem er einen oder mehrere vordefinierte Mikrowellenbestrahlungsparameter zur vordefinierten Mikrowellendesinfektionsbehandlung gegen eines oder mehrere Pathogene einstellt und in der Speichereinheit (112) speichert;
> wobei die elektronischen Steuereinheiten (111) eingerichtet sind, die Mikrowellenbestrahlungseinheit (12) basierend auf dem/den vordefinierten Mikrowellenbestrahlungsparameter(n), der/die in den Speichereinheiten (112) gespeichert ist/sind, derart zu betreiben, dass die Mikrowellenbestrahlungseinheit (12) die vordefinierte(n) Mikrowellendesinfektionsbehandlung(en) gegen das/die Pathogen(e) durchführt;
> wobei die Mikrowellenbestrahlungseinheit (12) eingerichtet ist, bei einer Betätigung durch die elektronische Steuereinheit (111), eine oder mehrere Mikrowellenbestrahlungen basierend auf den vordefinierten Mikrowellenbestrahlungsparametern durchzuführen;
> **dadurch gekennzeichnet, dass** die Mensch-Maschine-Schnittstelleneinheit (13) so eingerichtet ist, dass sie es dem Benutzer ermöglicht, für ein spezifisches Pathogen einen oder mehrere entsprechend vordefinierte Mikrowellenbestrahlungsparameter für eine vordefinierte Mikrowellendesinfektionsbehandlung einzustellen und in der Speichereinheit (112) zu speichern

2. Mikrowellendesinfektionssystem (1) gemäß Anspruch 1, wobei die Mensch-Maschine-Schnittstelleneinheit (13) es einem Benutzer ermöglicht, für jedes spezifische Pathogen aus einer Vielzahl von gegebenen Pathogenen einen oder mehrere vordefinierte Mikrowellenbestrahlungsparameter für eine vordefinierte Mikrowellendesinfektionsbehandlung einzustellen und in der Speichereinheit (112) zu speichern.

3. Mikrowellendesinfektionssystem (1) gemäß Anspruch 1 oder 2, wobei der/die jeweilige(n) vordefinierte(n) Mikrowellenbestrahlungsparameter, bezogen auf das spezifische Pathogen oder auf jedes Pathogen aus der Vielzahl von gegebenen Pathogenen, einen oder mehrere der folgenden Parameter umfasst/umfassen, die sich auf die Mikrowellendeaktivierung des spezifischen Pathogens oder eines Pathogens aus der Vielzahl von gegebenen Pathogenen bezieht/en:

- eine oder mehrere vordefinierte Wellenformen der Mikrowellenbestrahlung;
- eine oder mehrere vordefinierte Wellenlängen oder Frequenzen der Mikrowellenbestrahlung und/oder ein oder mehrere vordefinierte Wellenlängenbänder oder Frequenzen der Mikrowellenbestrahlung;
- einen oder mehrere vordefinierte Zeitsteuerungsparameter der Mikrowellenbestrahlung;
- eine oder mehrere vordefinierte Leistungen der Mikrowellenbestrahlung.

4. Mikrowellendesinfektionssystem (1) nach einem der Ansprüche 1 bis 3, wobei die Mensch-Maschine-Schnittstelleneinheit (13) eingerichtet ist, in der Speichereinheit (112) für das spezifische Pathogen oder für jedes Pathogen aus der Vielzahl der gegebenen Pathogene eine betreffende Bibliothek für die den/die entsprechenden vordefinierten Mikrowellenbestrahlungsparameter zu speichern.

5. Mikrowellendesinfektionssystem (1) nach einem der Ansprüche 1 bis 4, wobei die Mikrowellendesinfektionsvorrichtung (10) ferner einen oder mehrere Pathogendetektoren (15) umfasst, die dazu eingerichtet sind,

- das Vorhandensein des spezifischen Pathogens oder eines oder mehrerer Pathogene aus der Vielzahl der gegebenen Pathogene zu erkennen; und
- das Vorhandensein des spezifischen Pathogens oder eines oder mehrerer Pathogene aus der Vielzahl der gegebenen Pathogene an die elektronische Steuereinheit (111) zu signalisieren;

wobei die elektronische Steuereinheit (111) eingerichtet ist, die Mikrowellenbestrahlungseinheit, basierend auf den jeweils vordefinierten Mikrowellenbestrahlungsparametern, die in der Speichereinheit (112) für das/die spezifische/n, vom Pathogendetektor erkannte/n Pathogen/e oder das/die Pathogen(e) der Vielzahl von gegebenen Pathogenen gespeichert sind, zu betreiben.

6. Mikrowellendesinfektionssystem (1) nach einem der vorhergehenden Ansprüche, wobei die Mikrowellendesinfektionsvorrichtung (10) ferner einen oder mehrere Präsenzsensoren (14) umfasst, die eingerichtet sind,

- das Vorhandensein einer oder mehrerer Personen zu erkennen; und
- das Vorhandensein einer oder mehrerer Personen an die elektronische Steuereinheit (111) zu signalisieren;

wobei die elektronische Steuereinheit (111) dazu eingerichtet ist, die Mikrowellenbestrahlungseinheit (12) zu betreiben, wenn der/die Präsenzsensor(en) (14) das Vorhandensein einer oder mehrerer Personen erfasst/erfassen.

7. Mikrowellendesinfektionssystem (1) nach einem der vorhergehenden Ansprüche, wobei die Mikrowellendesinfektionsvorrichtung (10) ferner einen oder mehrere Umgebungssensoren (16) umfasst, die eingerichtet sind, betreffende Umgebungsüberwachungsdaten an die elektronische Steuereinheit (111) zu senden, und wobei die elektronische Steuereinheit (111) eingerichtet ist, den Betrieb der Mikrowellenbestrahlungseinheit (12) auch basierend auf den von dem/den Umgebungssensor/en erhaltenen Umgebungsüberwachungsdaten zu steuern.

8. Mikrowellendesinfektionssystem nach einem der vorhergehenden Ansprüche, wobei die Mensch-Maschine-Schnittstelleneinrichtung (13) über:

- eine externe und entweder über Kabel oder über eine drahtlose Verbindung mit der Mikrowellendesinfektionsvorrichtung (10) oder über
- Integration in die Mikrowellendesinfektionsvorrichtung (10)
realisiert ist.

9. Mikrowellendesinfektionssystem (1) nach einem der vorhergehenden Ansprüche, wobei die Mikrowellenbestrahlungseinheit (12)

- eine elektronische Signalerzeugungsvorrichtung (121);
- eine elektronische Signalverstärkungsvorrichtung (123); und
- eine Antenne (125)
umfasst.

10. Mikrowellendesinfektionssystem gemäß Anspruch 9, wobei die Mikrowellenbestrahlungseinheit (12) ferner eine Signalfiltervorrichtung (124) beinhaltet.

11. Mikrowellendesinfektionsverfahren basierend auf der Verwendung des Mikrowellendesinfektionssystems (1) nach einem der vorhergehenden Ansprüche, wobei das Mikrowellendesinfektionsverfahren

- die Durchführung von Mikrowellenbestrahlungstests an einem oder mehreren Pathogenen zur Bestimmung eines oder mehrerer Mikrowellenbestrahlungsparameter für eine oder mehrere Mikrowellendesinfektionsbehandlungen gegen das/die Pathogen(e);
- das Einstellen und Speichern des/der bestimmten Mikrowellenbestrahlungsparameter(s) in den Speichereinheiten (112) über die Mensch-Maschine-Schnittstelleneinheiten (13);
- die Anwendung der Mikrowellendesinfektionsbehandlung(en) gegen das/die Pathogen(e)
umfasst,

**dadurch gekennzeichnet, dass** ferner

- Mikrowellenbestrahlungstests an einem spezifischen Pathogen zur Bestimmung eines oder mehrerer betreffenden/r Mikrowellenbestrahlungsparameter zur Mikrowellendesinfektionsbehandlung gegen das/die spezifische(n) Pathogen(e) durchgeführt werden,
- der/die betreffenden Mikrowellenbestrahlungsparameter(s), der/die für das spezifische Pathogen bestimmt wurde(n), in den Speichereinheiten (112) über die Mensch-Maschine-Schnittstelleneinheiten (13) eingestellt und gespeichert werden und
- die Mikrowellendesinfektionsvorrichtung (10) zur Mikrowellendesinfektionsbehandlung(en) gegen das spezifische Pathogen angewendet wird.

12. Mikrowellendesinfektionsverfahren gemäß Anspruch 11, umfassend:

- die Durchführung von Mikrowellenbestrahlungstests an einer Vielzahl von gegebenen Pathogenen, um für jedes der gegebenen Pathogene einen oder mehrere Mikrowellenbestrahlungsparameter für die entsprechende/n Mikrowellendesinfektionsbehandlung/en gegen das genannte Pathogen der genannten Vielzahl von gegebenen Pathogenen zu bestimmen;
- das Einstellen und Speichern der für die gegebenen Pathogene bestimmten Mikrowellenbestrahlungsparameter in den Speichereinheiten (112) über die Mensch-Maschine-Schnittstelleneinheit (13); und
- Anwenden der Mikrowellendesinfektionsvorrichtung (10) zur Durchführung der Mikrowellenbehandlung der gegebenen Pathogene.

13. Mikrowellendesinfektionsverfahren gemäß Anspruch 11 oder 12, wobei der (die) jeweilige(n) Mikrowellenbestrahlungsparameter, der (die) für das spezifische Pathogen oder für jedes der gegebenen Pathogene bestimmt wurde(n), einen oder mehrere der folgenden Parameter zur Mikrowellendeaktivierung des spezifischen Pathogens oder des Pathogens aus der Vielzahl der gegebenen Pathogene umfasst/umfassen:

- eine oder mehrere Wellenformen der Mikrowellenbestrahlung;
- eine oder mehrere Wellenlängen oder Frequenzen der Mikrowellenbestrahlung und/oder ein oder mehrere Wellenlängenbänder oder Frequenzen der Mikrowellenbestrahlung;
- ein oder mehrere Zeitsteuerungsparameter der Mikrowellenbestrahlung;
- eine oder mehrere Leistungen der Mikrowellenbestrahlung.

**Revendications**

1. Système de désinfection par micro-ondes (1) configuré pour détruire ou désactiver en temps réel un ou plusieurs pathogènes au moyen d'une ou plusieurs irradiations de micro-ondes, dans lequel ledit système de désinfection par micro-ondes (1) comprend un dispositif de désinfection par micro-ondes (10) comportant :

- une section de commande (11) comportant des moyens de commande électroniques (111) et des moyens de stockage (112) ; et
- une section d'irradiation de micro-ondes (12) commandée par les moyens de commande électroniques (111) et configurée pour émettre des signaux micro-ondes ;
le système de désinfection par micro-ondes (1) comprenant en outre des moyens d'interface homme-machine (13) connectés à la section de commande (11) et configurés pour permettre à un utilisateur de configurer la section de commande (11) en établissant et stockant dans les moyens de stockage (112) un ou plusieurs paramètres d'irradiation de micro-ondes prédéfinis associés à un ou plusieurs traitements de désinfection par micro-ondes prédéfinis contre un ou plusieurs agents pathogènes ;

dans lequel les moyens de commande électroniques (111) sont configurés pour faire fonctionner la section d'irradiation de micro-ondes (12) sur la base du (des) paramètre(s) d'irradiation de micro-ondes prédéfini(s) stocké (s) dans les moyens de stockage (112) de telle sorte que ladite section d'irradiation de micro-ondes (12) réalise le(s) traitement(s) de désinfection par micro-ondes prédéfini(s) contre ledit (lesdits) agent(s) pathogène(s) ;

dans lequel la section d'irradiation de micro-ondes (12) est configurée pour, lorsqu'elle est actionnée par les moyens de commande électroniques (111), réaliser une ou plusieurs irradiations de micro-ondes sur la base dudit (desdits) paramètre(s) d'irradiation de micro-ondes prédéfini(s) ;

**caractérisé en ce que** les moyens d'interface homme-machine (13) sont configurés pour permettre à un utilisateur d'établir et de stocker dans les moyens de stockage (112), pour un pathogène spécifique, un ou plusieurs paramètres d'irradiation de micro-ondes prédéfinis respectifs associés à un ou plusieurs traitements de désinfection par micro-ondes prédéfinis respectifs contre ledit agent pathogène spécifique.

2. Système de désinfection par micro-ondes selon la revendication 1, dans lequel les moyens d'interface homme-machine (13) sont configurés pour permettre à un utilisateur d'établir et de stocker dans les moyens de stockage (112), pour chaque agent pathogène d'une pluralité d'agents pathogènes donnés, un ou plusieurs paramètres d'irradiation de micro-ondes prédéfinis respectifs associés à un ou plusieurs traitements de désinfection par micro-ondes prédéfinis respectifs contre ledit agent pathogène de ladite pluralité d'agents pathogènes donnés.

3. Système de désinfection par micro-ondes selon la revendication 1 ou 2, dans lequel le(s) paramètre(s) d'irradiation de micro-ondes prédéfini(s) respectif(s) associé(s) à l'agent pathogène spécifique ou à chaque agent pathogène de ladite pluralité d'agents pathogènes donnés comprend (comprennent) un ou plusieurs des paramètres suivants associés à une désactivation par micro-ondes dudit agent pathogène spécifique ou dudit agent pathogène de ladite pluralité d'agents pathogènes donnés :

- une ou plusieurs formes d'onde d'irradiation de micro-ondes prédéfinies ;
- une ou plusieurs longueurs d'onde ou fréquences prédéfinies d'irradiation de micro-ondes et/ou une ou plusieurs bandes prédéfinies de longueur d'onde ou de fréquence d'irradiation de micro-ondes ;
- un ou plusieurs paramètres de synchronisation d'irradiation de micro-ondes prédéfinis ;
- une ou plusieurs puissances d'irradiation de micro-ondes prédéfinies.

4. Système de désinfection par micro-ondes selon l'une quelconque des revendications 1 à 3, dans lequel les moyens d'interface homme-machine (13) sont configurés pour stocker dans les moyens de stockage (112), pour l'agent pathogène spécifique ou pour chaque agent pathogène de la pluralité d'agents pathogènes donnés, une banque respective contenant le(s) paramètre(s) d'irradiation de micro-ondes prédéfini(s) respectif(s).

5. Système de désinfection par micro-ondes selon l'une quelconque des revendications 1 à 4, dans lequel le dispositif de désinfection par micro-ondes (10) comprend en outre un ou plusieurs détecteurs d'agents pathogènes (15) configurés pour :

- détecter la présence de l'agent pathogène spécifique ou d'un ou plusieurs agents pathogènes parmi la pluralité d'agents pathogènes donnés ; et
- signaler la présence de l'agent pathogène spécifique ou d'un ou plusieurs agents pathogènes parmi la pluralité d'agents pathogènes donnés aux moyens de commande électroniques (111) ;

dans lequel les moyens de commande électroniques (111) sont configurés pour faire fonctionner la section d'irradiation de micro-ondes (12) sur la base du (des) paramètre(s) d'irradiation de micro-ondes prédéfini(s) respectif(s) stocké(s) dans les moyens de stockage (112) pour l'agent pathogène spécifique ou le(s) agent(s) pathogène(s) de la pluralité d'agents pathogènes donnés détectés par le (les) détecteur(s) d'agents pathogènes (15) .

6. Système de désinfection par micro-ondes selon l'une quelconque des revendications précédentes, dans lequel le dispositif de désinfection par micro-ondes (10) comprend en outre un ou plusieurs capteurs de présence (14) configurés pour :

- détecter la présence d'une ou plusieurs personnes ; et
- signaler la présence d'une ou plusieurs personnes aux moyens de commande électroniques (111) ;

dans lequel les moyens de commande électroniques (111) sont configurés pour faire fonctionner la section d'irradiation de micro-ondes (12) lorsque le (les) capteur(s) de présence (14) détecte(nt) la présence d'une ou plusieurs personnes.

7. Système de désinfection par micro-ondes selon l'une quelconque des revendications précédentes, dans lequel le dispositif de désinfection par micro-ondes (10) comprend en outre un ou plusieurs capteurs environnementaux (16) configurés pour envoyer des données de surveillance environnementale respectives aux moyens de commande électroniques (111), et dans lequel lesdits moyens de commande électroniques (111) sont configurés pour commander le fonctionnement de la section d'irradiation de micro-ondes (12) sur la base également des données de surveillance environnementale reçues du (des) capteur(s) environnemental(aux) (16).

8. Système de désinfection par micro-ondes selon l'une quelconque des revendications précédentes, dans lequel les moyens d'interface homme-machine (13) sont :

   • externes, et câblés ou connectés sans fil, au dispositif de désinfection par micro-ondes (10) ; ou
   • intégrés audit dispositif de désinfection par micro-ondes (10).

9. Système de désinfection par micro-ondes selon l'une quelconque des revendications précédentes, dans lequel la section d'irradiation de micro-ondes (12) comporte :

   • des moyens électroniques de génération de signal (121) ;
   • des moyens électroniques d'amplification de signal (123) ; et
   • une antenne (125).

10. Système de désinfection par micro-ondes selon la revendication 9, dans lequel la section d'irradiation de micro-ondes (12) comporte en outre des moyens de filtrage de signal (124).

11. Méthode de désinfection par micro-ondes basée sur l'utilisation du système de désinfection par micro-ondes (1) selon l'une quelconque des revendications précédentes, dans laquelle ladite méthode de désinfection par micro-ondes comprend :

   • la réalisation de tests par irradiation de micro-ondes sur un ou plusieurs agents pathogènes pour déterminer un ou plusieurs paramètres d'irradiation de micro-ondes associés à un ou plusieurs traitements de désinfection par micro-ondes contre ledit (lesdits) pathogène(s) ;
   • l'établissement et le stockage dans les moyens de stockage (112), au moyen des moyens d'interface homme-machine (13), le(s) paramètre(s) d'irradiation de micro-ondes déterminé(s) ;
   • l'utilisation du dispositif de désinfection par micro-ondes (10) pour réaliser le(s) traitement(s) de désinfection par micro-ondes contre le(s) agent(s) pathogène(s) ;

   **caractérisé en ce qu'**elle comprend en outre :

   • la réalisation de tests par irradiation de micro-ondes sur un agent pathogène spécifique pour déterminer un ou plusieurs paramètres d'irradiation de micro-ondes respectifs associés à un ou plusieurs traitements de désinfection par micro-ondes respectifs contre ledit agent pathogène spécifique ;
   • l'établissement et le stockage dans les moyens de stockage (112), au moyen des moyens d'interface homme-machine (13), du (des) paramètre(s) d'irradiation de micro-ondes respectif(s) déterminé(s) pour l'agent pathogène spécifique ; et
   • l'utilisation du dispositif de désinfection par micro-ondes (10) pour réaliser ledit (lesdits) traitement(s) de désinfection par micro-ondes contre ledit pathogène spécifique.

12. Méthode de désinfection par micro-ondes selon la revendication 11, comprenant :

   • la réalisation de tests par irradiation de micro-ondes sur une pluralité d'agents pathogènes donnés pour déterminer, pour chacun des agents pathogènes donnés, un ou plusieurs paramètres d'irradiation de micro-ondes respectifs associés à un ou plusieurs traitements de désinfection par micro-ondes respectifs contre ledit agent pathogène de ladite pluralité d'agents pathogènes donnés ;
   • l'établissement et le stockage dans les moyens de stockage (112), au moyen des moyens d'interface homme-machine (13), des paramètres d'irradiation de micro-ondes déterminés pour les agents pathogènes donnés ; et

• l'utilisation du dispositif de désinfection par micro-ondes (10) pour réaliser des traitements de désinfection par micro-ondes contre lesdits agents pathogènes donnés.

**13.** Méthode de désinfection par micro-ondes selon la revendication 11 ou 12, dans lequel le(s) paramètre(s) d'irradiation de micro-ondes respectif(s) déterminé(s) pour l'agent pathogène spécifique ou pour chacun des agents pathogènes donnés comprend (comprennent) un ou plusieurs des paramètres suivants associés à la désactivation par micro-ondes dudit agent pathogène spécifique ou dudit agent pathogène de la pluralité d'agents pathogènes donnés :

• une ou plusieurs formes d'onde d'irradiation de micro-ondes ;
• une ou plusieurs longueurs d'onde ou fréquences d'irradiation de micro-ondes et/ou une ou plusieurs bandes de longueur d'onde ou de fréquence d'irradiation de micro-ondes ;
• un ou plusieurs paramètres de synchronisation d'irradiation de micro-ondes ;
• une ou plusieurs puissances d'irradiation de micro-ondes.

FIG. 1

10A

FIG. 2

FIG. 3

FIG. 4

FIG. 5A

FIG. 5B

FIG 6

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 10561751 B1

### Non-patent literature cited in the description

- **REZA HAJIAN et al.** Detection of unamplified target genes via CRISPR-Cas9 immobilized on a graphene field-effect transistor. *Nature Biomedical Engineering,* 25 March 2019, vol. 3, 427-437
- **YANG SC et al.** Efficient Structure Resonance Energy Transfer from Microwaves to Confined Acoustic Vibrations in Viruses. *Scientific Reports,* December 2015, vol. 5, 18030
- ICNRIP Guidelines for Limiting Exposure to Time-Varying Electric, Magnetic and Electromagnetic Fields (Up to 300 GHz). *Health Physics,* 1998, vol. 74 (4), 494-522
- Raccomandazioni ad interim sulla sanificazione di strutture non sanitarie nell'attuale emergenza COVID-19: superfici, ambienti interni e abbigliamento. *Report by the Istituto Superiore di Sanità (ISS),* 15 May 2020
- **A. BARBORA ; R. MINNES.** *Targeted antiviral treatment using Non-ionizing Radiation Therapy for SARS-CoV-2 and viral pandemics preparedness: Technique, methods and practical notes for Clinical Application,* August 2020
- **C. WANG et al.** Airborne disinfection using microwave-based technology: Energy efficient and distinct inactivation mechanism compared with waterborne disinfection. *Journal of Aerosol Science,* 2019, vol. 137